# EUROPEAN PATENT APPLICATION

(11) **EP 4 792 876 A1**
(43) Date of publication of application: **19.08.2026**
(21) Application number: 25220879.8
(22) Date of filing: 04.12.2025
(51) Int. Cl.: A61N 5/10

(54) **TREATMENT PLANNING AND DELIVERY**

(30) Priority: 04.12.2024 GB 202417816
(71) Applicant: Elekta Limited, Crawley, Sussex RH10 9RR (GB)
(72) Inventor: SCHRECK, Oliver, Crawley, RH10 9BL (GB)
(74) Representative: Woodhill, Matthew James

(57) **Abstract**

There is provided a computer-implemented method for determining a radiotherapy treatment plan to be carried out using a radiotherapy device comprising a radiotherapy beam source mounted to a gantry, the radiotherapy beam source configured to irradiate a subject with a radiotherapy beam. The method comprises receiving data indicating movement of a target region of a patient over a period of time, identifying, based on the received data, a plurality of discrete positions of the target region, each discrete position of the plurality of positions corresponding to a different time within the period of time, and determining, for each of the plurality of discrete positions, a respective primary treatment sub-plan. Each primary treatment sub-plan comprises a respective set of radiation delivery parameters defining radiotherapy delivery to the patient, wherein the radiation delivery parameters include one or more of: gantry rotation angle of delivery; gantry tilt angle of delivery; radiotherapy beam shape per rotation angle and/or tile angle of delivery; radiotherapy beam energy per rotation angle and/or tilt angle of delivery; the duration of application of the radiotherapy beam per rotation angle and/or tilt angle of delivery; and patient position per gantry rotation angle and/or tilt angle of delivery. The set of radiation delivery parameters is determined for each primary treatment sub-plan based on the respective discrete position of the target region.

## Description

This disclosure relates to systems and methods for carrying radiation therapy, and in particular for carrying out radiation therapy on moving targets.

### Background

Radiotherapy involves the use of ionising radiation, such as X-rays, to treat a human or animal body. Radiotherapy is commonly used to treat tumours within the body of a patient or subject. In such treatments, ionising radiation is used to irradiate, and thus destroy or damage, cells which form part of the tumour.

In radiotherapy treatment, it is desirable to deliver a prescribed dose of radiation to a target region, e.g., a tumour, of a subject and to limit irradiation of other parts of the subject, e.g., healthy tissue and organs at risk (OARs).

The treatment planning procedure typically involves obtaining one or more medical images, such as a CT image of the patient, and segmenting them to identify the target region and OARs near the target region. The segmentation process can be performed manually or using auto-segmentation techniques. The clinician determines radiation treatment parameters, for example by prescribing a radiation dose to be delivered to the target region and maximum doses to be delivered to the various OARs. The treatment planning procedure then involves optimizing various radiation delivery variables to meet the prescribed radiation treatment parameters, for example determining the number of sessions (or 'fractions') over which radiotherapy should be conducted, the angles at which the radiation beam should be applied during each fraction (including gantry rotation angles and treatment head tilt angles), at what beam energy, the duration of application of the radiation beams at these angles, and the beam shape(s) at each angle of delivery. The clinician can be assisted by software during some or all of these steps. This aspect of treatment planning, conducted in advance of the patient's treatment, can be described as "offline" treatment planning.

However, the characteristics of the tumour, such as its shape, size, and location relative to a radiotherapy system, will change over the course of the multiple fractions of treatment. Similarly, other patient information and characteristics of the patient's anatomy can change between fractions. Accordingly, online adaptive radiotherapy techniques may be used to update and re-optimise the radiotherapy treatment plan immediately prior to the patient's treatment. According to online adaptive radiotherapy techniques, the patient is imaged again immediately prior to treatment, and the treatment plan for that day's fraction may be adjusted / reoptimized according to the latest available medical images. Online adaptive radiation therapy techniques therefore allow inter-fraction anatomical changes to be taken into account.

In addition to changes that may occur over multiple fractions of treatment, changes to the shape, size, location of a tumour or OAR may also occur during a single fraction of treatment. Motion of the subject can cause a decreased dose to be applied to the target region and/or an increased dose to be applied to the surrounding healthy tissue. There are various physiological motions that can contribute to a total motion of a subject. Gross or large-scale movements of a subject may include shifting position, coughing or sneezing. The subject may also undergo cyclical, physiological movement. For example, the target region is likely to undergo respiratory motion due to the patient's breathing cycle. To address this motion, known techniques include monitoring a location and/or movement of the subject and gating the treatment beam such that radiation is applied only when the subject/target region is in a desired location and not when the subject/target region is in a suboptimal location. In other words, radiation may be applied or not applied (i.e., the therapeutic radiation beam may be 'gated') based on sensed movements or locations of the subject. Other known techniques for addressing movement of the subject include training a patient's breathing or asking the patient to hold their breath during radiotherapy treatment. Known techniques for addressing gross or large-scale movements of a subject also currently include gating the treatment beam such that radiation is not applied to healthy tissue as a result of the large-scale movement.

Recently, efforts have been made to develop and employ inline (or 'real-time') adaptive radiation therapy. In part, these efforts have been enabled by the development of radiotherapy machines comprising magnetic resonance (MR) imaging capabilities, for example MR-linac devices. At a highlevel, the aim of real-time adaptive radiation therapy techniques is to monitor the position of the patient's anatomy, for example the target region and OARs, during treatment, and update the treatment in real-time to take into account movement of the tumour and the surrounding OARs.

However, technical difficulties exist when trying to employ true real-time adaptive radiation therapy. A greater amount of automation and autonomous decision making is required compared to traditional purely offline or online techniques. It is important to ensure that treatment is updated within the bounds of regulatory QA requirements, and that appropriate oversight by the clinician is maintained. In addition, continuous tracking of the tumour and other anatomical features in order to update and re-optimise the radiation delivery variables, in real-time, in response to detected tumour movement and deformation creates a huge processing resource constraint. In other words, current real-time adaptive techniques require a huge amount of processing power. Any delay between image acquisition and the updating of delivery variables should be minimised to ensure that treatment is optimised, and for this reason it is important for real-time techniques to reduce latency as much as possible despite the large computational burden required to properly implement real-time adaptive treatment methods.

The present invention seeks to address these and other disadvantages encountered in the prior art.

### Summary

According to a first aspect, there is provided a computer-implemented method for determining a radiotherapy treatment plan to be carried out using a radiotherapy device comprising a radiotherapy beam source mounted to a gantry. The radiotherapy beam source is configured to irradiate a subject with a radiotherapy beam. The method comprises receiving data indicating movement of a target region of a patient over a period of time, identifying, based on the received data, a plurality of discrete positions of the target region, each discrete position of the plurality of positions corresponding to a different time within the period of time, and determining, for each of the plurality of discrete positions, a respective primary treatment sub-plan. Each primary treatment subplan comprises a respective set of radiation delivery parameters defining radiotherapy delivery to the patient. The radiation delivery parameters include one or more of: gantry rotation angle of delivery; gantry tilt angle of delivery; radiotherapy beam shape per rotation angle and/or tile angle of delivery; radiotherapy beam energy per rotation angle and/or tilt angle of delivery; the duration of application of the radiotherapy beam per rotation angle and/or tilt angle of delivery; and patient position per gantry rotation angle and/or tilt angle of delivery. The set of radiation delivery parameters is determined for each primary treatment sub-plan based on the respective discrete position of the target region.

In some embodiments, the method further comprises creating a primary look-up table that associates one or more locations of the target region over the period of time with the respective primary treatment sub-plan.

In some embodiments, the plurality of discrete positions of the target region are based on a location of centre of mass of the target region.

In some embodiments, the target region comprises healthy tissue.

In some embodiments, the set of radiation delivery parameters determined for each primary treatment sub-plan is further based on a shape of the target region at the respective discrete position.

In some embodiments, the method further comprises: determining a movement boundary volume around the target region, wherein the movement boundary volume is a volume of space encapsulating each of the plurality of discrete positions of the target region; identifying a plurality of locations within the movement boundary volume that do not correspond to any of the plurality of discrete positions of the target region; and determining, for each identified location, a respective secondary treatment sub-plan. Each secondary treatment sub-plan comprises a respective set of radiation delivery parameters defining radiotherapy delivery to the patient, and wherein the set of radiation delivery parameters is determined for each secondary sub-plan based on centre of mass of the target region being located at the corresponding location within the movement boundary volume.

In some embodiments, the method further comprises creating a secondary look-up table that associates the one or more locations within the movement boundary volume that do not correspond to the discrete positions of the target region with the respective secondary treatment sub-plan.

In some embodiments, the size of the boundary movement volume is determined by one or more of: a size of the target region; a shape of the target region; a type of healthy tissue adjacent to the target region; a proximity of the target region to adjacent healthy tissue; and a shape or size of the radiotherapy beam.

In some embodiments, the movement of the target region over the period of time defines a motion path and wherein each primary sub-plan corresponds to a different position along the motion path.

In some embodiments, each secondary sub-plan corresponds to a location within the movement boundary volume that is not on the motion path.

In some embodiments, the received data comprises a plurality of image frames, wherein each frame identifies one of the discrete positions of the plurality of discrete positions of the target region, and wherein each primary sub-plan corresponds to the discrete position of target region in a respective one of the plurality of frames.

In some embodiments, determining the primary treatment sub-plans comprises determining a primary treatment sub-plan for every Nth frame of the plurality of frames, where N is a predetermined frame interval number.

In some embodiments, determining the plurality of primary treatment sub-plans comprises determining a primary treatment sub-plan for each frame of a subset of the plurality of frames, wherein the subset of frames are selected such that a displacement of the position of the centre of mass of the target region between successive frames in the subset is greater than or equal to a threshold displacement. Optionally, the threshold displacement amount is 10mm or less, optionally 5mm or less, optionally 2mm or less, optionally 1mm.

The threshold displacement may represent an amount of movement of the target region away from any discrete position of the target region for which it is acceptable to provide radiotherapy treatment according to a primary treatment sub-plan that corresponds to the given discrete position. The amount of movement of the target region may be determined by one or more factors, including one or more of: a size of the target region, a shape of the target region, a type of healthy tissue adjacent to the target region, a proximity of the target region to the adjacent healthy tissue, a shape or size of the radiotherapy beam.

According to a second aspect, there is provided a computer-implemented method for determining a radiotherapy treatment plan to be carried out using a radiotherapy device, wherein the radiotherapy device comprises a radiotherapy beam source mounted to a gantry, wherein the radiotherapy beam source configured to irradiate a subject with a radiotherapy beam. The method comprises receiving data indicating position information for a target region of a patient; determining, based on the received data, a current position of the target region; and selecting, based on the current position of the target region, a treatment sub-plan from a plurality of predetermined treatment sub plans, wherein each of the predetermined treatment sub-plans corresponds to a different position of the target region and wherein the selected treatment sub-plan corresponds to the determined current position of the target region. Each sub-plan comprises a respective set of radiation delivery parameters defining radiotherapy delivery to the patient, wherein the radiation delivery parameters include one or more of: gantry rotation angle of delivery; gantry tilt angle of delivery; radiotherapy beam shape per rotation angle and/or tile angle of delivery; radiotherapy beam energy per rotation angle and/or tilt angle of delivery; the duration of application of the radiotherapy beam per rotation angle and/or tilt angle of delivery; and patient position per gantry rotation angle and/or tilt angle of delivery.

In some embodiments, the method further comprises sending a control signal to the radiotherapy device based on the selected sub-plan to cause the radiotherapy device to deliver radiotherapy to the patient in accordance with the set of radiation delivery parameters of the selected treatment sub-plan.

In some embodiments, selecting the treatment sub-plan comprises accessing one or more look-up tables that store an association between target region positions and corresponding predetermined treatment sub-plans that were determined according to the respective target region position, and selecting a predetermined treatment sub-plan from the look-up table based on the associated target region position that matches the determined target region position.

In some embodiments, the method further comprises receiving updated data indicating position information for the target region of the patient, determining, based on the updated data, an updated position of the target region, and selecting, based on the updated position of the target region, a second treatment sub-plan from the plurality of predetermined treatment sub-plans that corresponds to the updated position of the target region.

In some embodiments, the method further comprises sending an updated control signal to the radiotherapy device based on the selected second sub-plan to cause the radiotherapy device to deliver radiotherapy to the patient in accordance with the radiation delivery parameters of the second sub-plan.

In some embodiments, selecting a treatment sub-plan from a plurality of predetermined treatment sub plans comprises determining whether the current position of the target region corresponds to a predetermined position of the target region identified during a treatment planning phase, responsive to determining that the current position of the target region corresponds to any one of a plurality of predetermined positions of the target region identified during a treatment planning phase: selecting a treatment sub-plan from a first set of treatment sub-plans, the first set of treatment sub-plans each corresponding to a different predetermined position of the target region, wherein the selected treatment sub-plan corresponds to the current position of the target region, and responsive to determining that the current position of the target region does not correspond to one of the plurality of predetermined position of the target region, determining that the current position of the region corresponds to a location within a predetermined volume of space encapsulating the plurality of predetermined positions; and selecting a treatment sub-plan from a second set of treatment sub-plans, the second set of treatment sub-plans each corresponding to a different location within the predetermined volume of space, wherein the selected treatment subplan corresponds to the current position of the target region.

### Figures

Specific embodiments are now described, by way of example only, with reference to the drawings, in which:
Figure 1 depicts a radiotherapy device or apparatus according to the present disclosure;
Figure 2 depicts a motion cycle of a target region;
Figure 3a depicts a movement boundary volume encapsulating a motion cycle of a target region according to the present disclosure;
Figure 3b depicts a shifted motion cycle of a target region according to the present disclosure;
Figure 4 is a flowchart for a method of treatment planning according to the present disclosure;
Figure 5 is a flowchart for a method of treatment planning according to the present disclosure;
Figure 6 is a flowchart for a method of selecting a radiotherapy treatment plan according to the present disclosure;
Figure 7 illustrates a block diagram of one implementation of a radiotherapy system according to the present disclosure; and
Figure 8 illustrates a computer program product storing instructions according to the present disclosure.

### Detailed Description

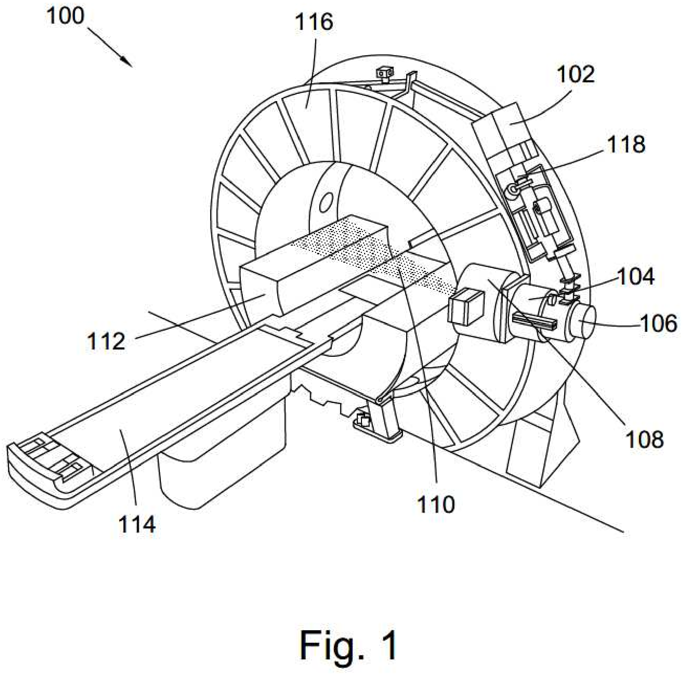

Figure 1 depicts a radiotherapy device suitable for delivering, and configured to deliver, a beam of radiation to a patient during radiotherapy treatment. The device and its constituent components will be described generally for the purpose of providing useful accompanying information for the present invention. The device depicted in figure 1 is in accordance with the present disclosure and is suitable for use with the disclosed systems and apparatuses. While the device in figure 1 is an MR-linac, the implementations of the present disclosure may be any radiotherapy device, for example a linac device.

The device 100 depicted in figure 1 is an MR-linac. The device 100 comprises both MR imaging apparatus 112 and radiotherapy (RT) apparatus which may comprise a linac device. The MR imaging apparatus 112 is shown in cross-section in the diagram. In operation, the MR scanner produces MR images of the patient, and the linac device produces and shapes a beam of radiation and directs it toward a target region within a patient's body in accordance with a radiotherapy treatment plan. The depicted device does not have the usual 'housing' which would cover the MR imaging apparatus 112 and RT apparatus in a commercial setting such as a hospital.

The MR-linac device depicted in figure 1 comprises a source of radiofrequency waves 102, a waveguide 104, a source of electrons 106, a source of radiation 106, a collimator 108 such as a multi-leaf collimator configured to collimate and shape the beam, MR imaging apparatus 112, and a patient support surface 114. In use, the device would also comprise a housing (not shown) which, together with the ring-shaped gantry, defines a bore. The moveable support surface 114 can be used to move a patient, or other subject, into the bore when an MR scan and/or when radiotherapy is to commence. The MR imaging apparatus 112, RT apparatus, and a subject support surface actuator are communicatively coupled to a controller or processor. The controller is also communicatively coupled to a memory device comprising computer-executable instructions which may be executed by the controller.

The RT apparatus comprises a source of radiation and a radiation detector (not shown). Typically, the radiation detector is positioned diametrically opposed to the radiation source. The radiation detector is suitable for, and configured to, produce radiation intensity data. In particular, the radiation detector is positioned and configured to detect the intensity of radiation which has passed through the subject. The radiation detector may also be described as radiation detecting means, and may form part of a portal imaging system.

The radiation source may comprise a beam generation system. For a linac, the beam generation system may comprise a source of RF energy 102, an electron gun 106, and a waveguide 104. The radiation source is attached to the rotatable gantry 116 so as to rotate with the gantry 116. In this way, the radiation source is rotatable around the patient so that the treatment beam 110 can be applied from different angles around the gantry 116. In a preferred implementation, the gantry is continuously rotatable. In other words, the gantry can be rotated by 360 degrees around the patient, and in fact can continue to be rotated past 360 degrees. The gantry may be ring-shaped. In other words, the gantry may be a ring-gantry.

The source 102 of radiofrequency waves, such as a magnetron, is configured to produce radiofrequency waves. The source 102 of radiofrequency waves is coupled to the waveguide 104 via circulator 118, and is configured to pulse radiofrequency waves into the waveguide 104. Radiofrequency waves may pass from the source 102 of radiofrequency waves through an RF input window and into an RF input connecting pipe or tube. A source of electrons 106, such as an electron gun, is also coupled to the waveguide 104 and is configured to inject electrons into the waveguide 104. In the electron gun 106, electrons are thermionically emitted from a cathode filament as the filament is heated. The temperature of the filament controls the number of electrons injected. The injection of electrons into the waveguide 104 is synchronised with the pumping of the radiofrequency waves into the waveguide 104. The design and operation of the radiofrequency wave source 102, electron source and the waveguide 104 is such that the radiofrequency waves accelerate the electrons to very high energies as the electrons propagate through the waveguide 104.

The design of the waveguide 104 depends on whether the linac accelerates the electrons using a standing wave or travelling wave, though the waveguide typically comprises a series of cells or cavities, each cavity connected by a hole or 'iris' through which the electron beam may pass. The cavities are coupled in order that a suitable electric field pattern is produced which accelerates electrons propagating through the waveguide 104. As the electrons are accelerated in the waveguide 104, the electron beam path is controlled by a suitable arrangement of steering magnets, or steering coils, which surround the waveguide 104. The arrangement of steering magnets may comprise, for example, two sets of quadrupole magnets.

Once the electrons have been accelerated, they may pass into a flight tube. The flight tube may be connected to the waveguide by a connecting tube. This connecting tube or connecting structure may be called a drift tube. The electrons travel toward a heavy metal target which may comprise, for example, tungsten. Whilst the electrons travel through the flight tube, an arrangement of focusing magnets act to direct and focus the beam on the target.

To ensure that propagation of the electrons is not impeded as the electron beam travels toward the target, the waveguide 104 is evacuated using a vacuum system comprising a vacuum pump or an arrangement of vacuum pumps. The pump system is capable of producing ultra-high vacuum (UHV) conditions in the waveguide 104 and in the flight tube. The vacuum system also ensures UHV conditions in the electron gun. Electrons can be accelerated to speeds approaching the speed of light in the evacuated waveguide 104.

The source of radiation is configured to direct a beam 110 of therapeutic radiation toward a patient positioned on the patient support surface 114. The source of radiation may comprise a heavy metal target toward which the high energy electrons exiting the waveguide are directed. When the electrons strike the target, X-rays are produced in a variety of directions. A primary collimator may block X-rays travelling in certain directions and pass only forward travelling X-rays to produce a treatment beam 110. The X-rays may be filtered and may pass through one or more ion chambers for dose measuring. The beam can be shaped in various ways by beam-shaping apparatus, for example by using a multi-leaf collimator 108, before it passes into the patient as part of radiotherapy treatment.

The source of radiation may be configured to direct a beam 110 of therapeutic radiation toward a patient in both a coplanar configuration, (where the beam of radiation is generally normal to the rotation axis of the rotatable gantry 116), and a non-coplanar configuration (where the beam of radiation is generally directed at an oblique angle relative to the rotation axis of the rotatable gantry 116). To switch between the coplanar and non-coplanar configurations, the radiation treatment head (including at least the collimator 108) may move between a coplanar configuration as depicted in Figure 1 to a tilted configuration, where the radiation treatment head is no longer generally in the same rotation plane as the gantry and is instead extended array from the gantry in an axial direction (i.e. parallel to the rotation axis of the gantry 116).

In some implementations, the source of radiation is configured to emit either an X-ray beam or an electron particle beam. Such implementations allow the device to provide electron beam therapy, i.e., a type of external beam therapy where electrons, rather than X-rays, are directed toward the target region. It is possible to 'swap' between a first mode in which X-rays are emitted and a second mode in which electrons are emitted by adjusting the components of the linac. In essence, it is possible to swap between the first and second mode by moving the heavy metal target in or out of the electron beam path and replacing it with a so-called 'electron window'. The electron window is substantially transparent to electrons and allows electrons to exit the flight tube.

The subject or patient support surface 114 is configured to move between a first position substantially outside the bore, and a second position substantially inside the bore. In the first position, a patient or subject can mount the patient support surface 114. The support surface 114, and patient, can then be moved inside the bore, to the second position, in order for the patient to be imaged by the MR imaging apparatus 112 and/or imaged or treated using the RT apparatus. The movement of the patient support surface is effected and controlled by a subject support surface actuator, which may be described as an actuation mechanism. The actuation mechanism is configured to move the subject support surface in a direction parallel to, and defined by, the central axis of the bore. The terms subject and patient are used interchangeably herein such that the subject support surface can also be described as a patient support surface. The subject support surface may also be referred to as a moveable or adjustable couch or table. More generally, the patient support surface 114 may be moveable in up to 6 degrees of freedom, for example in a direction parallel to the rotation axis or in a direction perpendicular to the rotation axis. The patient support surface may also be rotatable about one or more axes.

The radiotherapy apparatus / device depicted in figure 1 also comprises MR imaging apparatus 112. The MR imaging apparatus 112 is configured to obtain images of a subject positioned, i.e., located, on the subject support surface 114. The MR imaging apparatus 112 may also be referred to as the MR imager. The MR imaging apparatus 112 may be a conventional MR imaging apparatus operating in a known manner to obtain MR data, for example MR images. The skilled person will appreciate that such a MR imaging apparatus 112 may comprise a primary magnet, one or more gradient coils, one or more receive coils, and an RF pulse applicator. The operation of the MR imaging apparatus is controlled by the controller.

The controller is a computer, processor, or other processing apparatus. The controller may be formed by several discrete processors; for example, the controller may comprise an MR imaging apparatus processor, which controls the MR imaging apparatus 110; an RT apparatus processor, which controls the operation of the RT apparatus; and a subject support surface processor which controls the operation and actuation of the subject support surface. The controller is communicatively coupled to a memory, e.g., a computer readable medium.

The linac device also comprises several other components and systems as will be understood by the skilled person. For example, in order to ensure the linac does not leak radiation, appropriate shielding is also provided.

### Target region motion tracking

### Primary treatment sub-plans

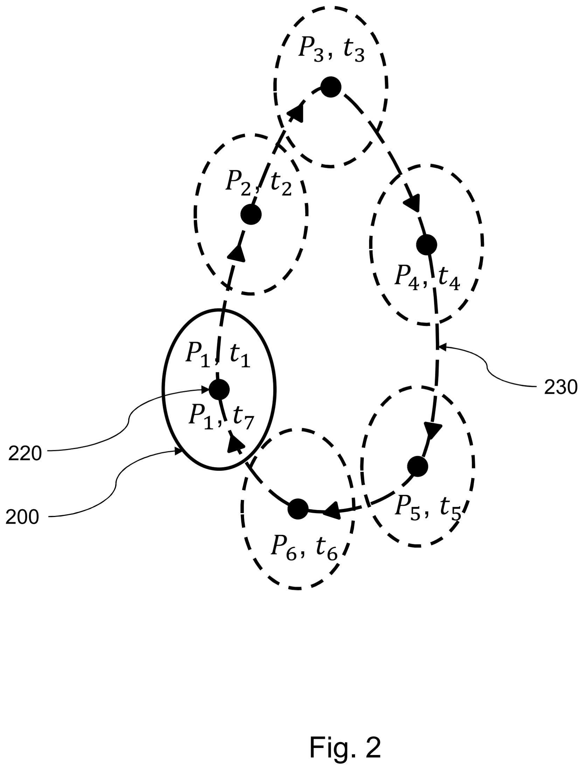

With reference to Figure 2, a schematic image (not to scale) illustrating movement of a target region 200 (e.g., a tumour, or an organ at risk, OAR) over a period of time is shown. As discussed above, locations within a patient's body, in particular target regions such as tumours or an organ at risk (OAR) may move within a patient's body over a period of time, for example due to the patient's breathing or other internal movement. As with the particular example shown in Figure 2, the movement may be cyclical and periodic, especially in the case of movement of a target region due to a breathing cycle. In such examples, the position of the target region may follow a cycle over a time period (e.g., corresponding to a respiratory rate of the patient). Whilst the exemplary schematic of Figure 2 is two-dimensional, it would be appreciated that movement of the target region may in practice be in one, two or three spatial dimensions.

In more detail, Figure 2 illustrates a target region 200 at a plurality of different discrete positions (e.g., *P*₁,*P*₂,*P*₃ and so on) labelled accordingly. Each discrete position corresponds to a respective point in time (*t*₁,*t*₂,*t*₃ and so on), such that at time *t*₁, the target region 200 is at position *P*₁. Each discrete position P of the target region may be defined in three-dimensional coordinates relative to a fixed reference frame (e.g., a reference frame fixed to a radiotherapy system). The discrete positions and corresponding times for the target region may be stored in a computer-readable medium as four-dimensional vectors, e.g. (*x*₁,*y*₁,*z*₁,*t*₁), (*x*₂,*y*_{2,}*z*₂,*t*₂)... (*x*₇,*y*₇,*z*₇,*t*₇). The position coordinates for each discrete position may be defined with respect to a centroid 220 of the target region 200, which may correspond to the centre of mass of the target region 200. In other words, the position coordinates (*xₙ,yₙ,zₙ*) (where *n* is an index corresponding to time *tₙ*) of the target region at each discrete position may actually refer to the position coordinates of the centroid (e.g. centre of mass) of the target region. In general, any reference made throughout the present disclosure to a position of the target region may refer to the position of the centroid or centre of mass of the target region.

In this particular example, the figure comprises six discrete positions of the target region 200 over a movement cycle, each position corresponding to a respective point in time. In more detail, the target region may continuously move from position *P*₁ at time *t*₁ to position *P*₂ at time *t*₂, then to position *P*₃ and so on, until the cycle is completed as the target region moves from position *P*₆ at time *t*₆ back to the initial position *P*₁ at time *t*₇. The periodic cycle may then repeat as the patient continues to breathe, or as a result of whichever anatomical process is causing the cyclical movement of the target region.

The discrete positions *P*₁ ... *P*₆ therefore represent the movement cycle of the target region over the time period *T* = *t*₇ - *t*₁, in which the target region continuously moves with the patient's body. Whilst in this illustrative example, six discrete positions of the target region are illustrated, it would be appreciated that the movement cycle of the target region over the time period T may be represented by any number of discrete positions *Pₘ* identified at a corresponding number of different times *tₘ* , where *m* could be up to 10, optionally up to 100, optionally up to 1000 or more or any suitable number in between.

The continuous movement of the centre of the target region over the period of time from *t*₁ to*t*₇ defines a motion path which is depicted as dashed line 230 in Figure 2. This line generally represents the path followed by the centre 220 of the target region has the target region moves between time *t*₁ and *t*₇, which can also be considered as the expected motion path of the target region over any given cycle (e.g. breathing cycle). Each discrete position of the (centre of the) target region captured from data (e.g., CT, MRI image information, or surface-guided techniques) generally lies on the motion path 230 and corresponds to a different location along the motion path.

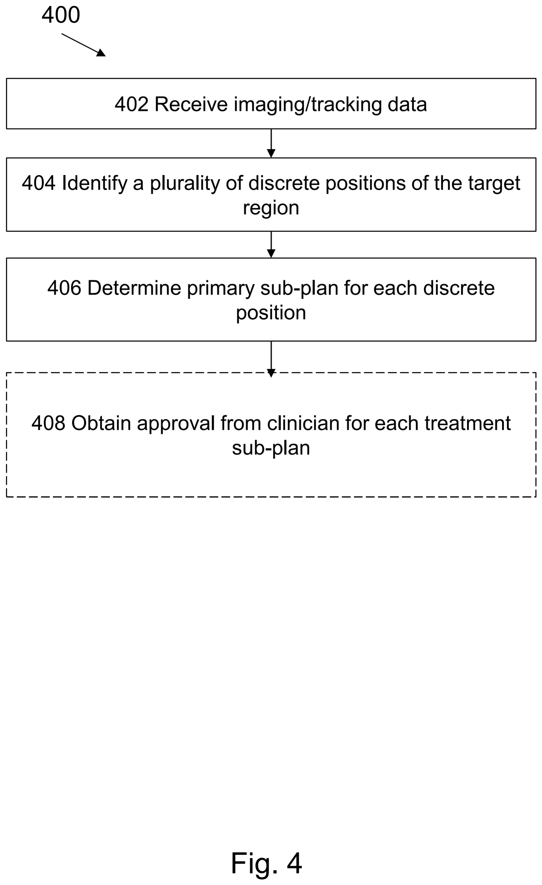

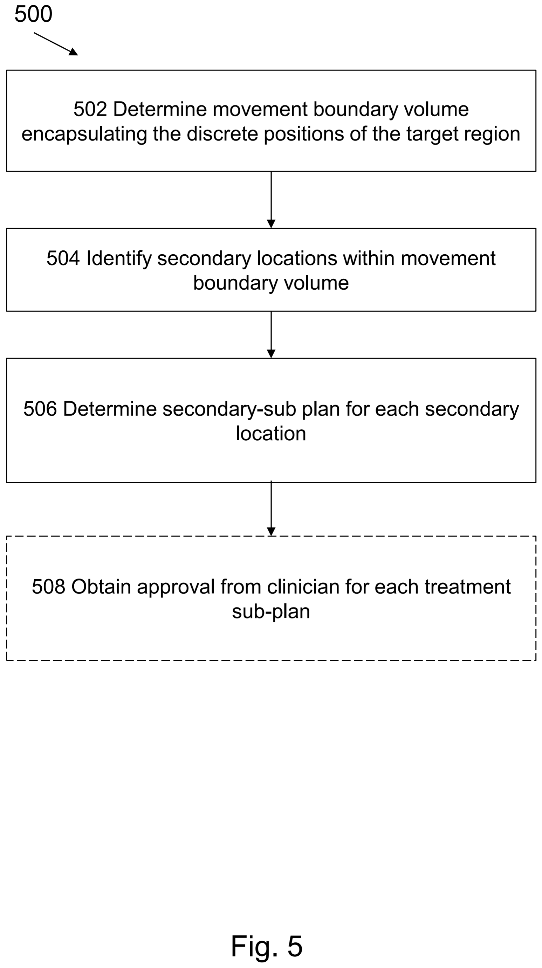

As described in more detail below, the movement of the target region may be determined by imaging the target region at a time before radiotherapy treatment takes place, i.e., in a pre-treatment phase (see discussion with respect to Figures 4 and 5 below). The target region may be imaged using imaging techniques such as, CBCT, PET, 4D CT, or any other suitable imaging technique apparent to the skilled person. Alternatively, the position of the target region may be determined using other techniques, such as surface-guided radiation therapy (SGRT)-based techniques that use external cameras to visually track external movement of a patient's body. As would be appreciated by the skilled person, visual tracking of the patient in this manner can be used to infer to locations and/or motions of the internal anatomy and in particular internal target regions of the patient. One example of a surface-guided technique that can be used to infer the internal position/movement of the target region involves one or more time-of-flight cameras that are used to measure the distance between the camera and a plurality of points on the patient based on the "time-of-flight" of the light signal. More generally, tracking the surface of the patient to infer the internal positioning and movement of target regions may be achieved using any suitable ranging technique apparent to the skilled person.

The target region 200 may be imaged (or otherwise tracked using surface-guided techniques) over a period of time (e.g., at least over a time period *T* of a single cycle of the cyclical movement) to track and capture the full cycle of movement of the target region. In the context of imaging the target region, the resulting image data comprises a plurality of image frames, each frame corresponding to a different time *t* within the period of time *T* over which the target region was imaged, where each frame images the target region at a different respective position *P*. In the context of tracking the target region using surface guided techniques, the resulting tracking data can be processed using known techniques to obtain a plurality of discrete positions *P* of the target region, each corresponding to a different time *t* within the period of time *T* over which the patient's surface was imaged. The skilled person would be readily aware of various systems and methods for tracking the shape, size and location of a target region within a patient during a pre-treatment phase of radiotherapy where a radiotherapy treatment plan is devised.

As described in more detail below with reference to Figure 4, a primary radiotherapy treatment subplan may be determined for each of one or more of the identified discrete positions (e.g., from the frames of the image data). During radiotherapy treatment, the position of the target region may be tracked (using imaging or surface-guided techniques) as it moves along the expected movement cycle (i.e., the motion path 230). At any given position of the target region, radiotherapeutic treatment can continually be delivered according to one of the primary sub-plans that corresponds to the given position. The sub-plan according to which treatment is delivered can therefore change as the target region moves between positions P.

### Secondary treatment sub-plans

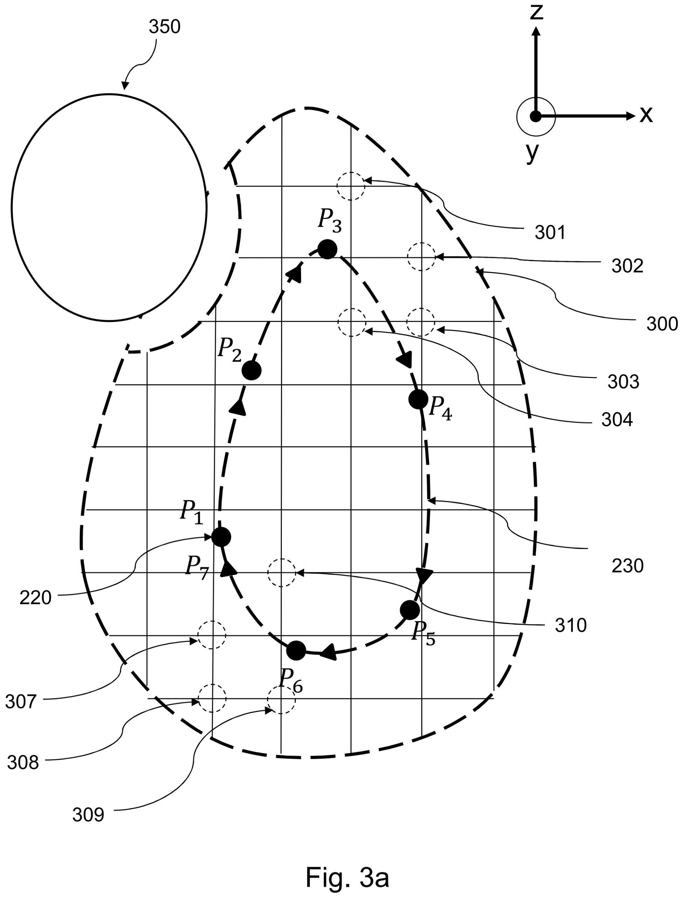

Referring to Figure 3a, the plurality of discrete positions *P*₁ ... *P*₇ of the centre of mass of the target region 200 are shown with the motion path 230, in addition to a movement boundary volume 300 that encapsulates (i.e. completely contains) each of the discrete positions *P*₁ ... *P*₇. The movement boundary volume 300 defines a plurality of additional discrete locations, outside of the plurality of discrete positions *P*₁ ... *P*₇, for which corresponding secondary treatment sub-plans are determined.

Each secondary treatment sub-plan is determined according to the assumption that the target region (e.g., the centre of mass of the target region) is located at the corresponding discrete location within the movement boundary volume. As such, if the target region position deviates from the motion path 230 but remains within the movement boundary volume, it is still possible to deliver radiation to the target region according to one of the secondary sub-plans that corresponds to the position of the target region within the movement boundary volume 300.

The use of secondary treatment sub-plans for discrete locations within a movement boundary volume outside of the expected motion path 230 of the target region contrasts existing techniques which require the treatment beam to be gated if the target region moves away from an expected position. By avoiding the need to gate the treatment beam due to an unexpected movement or shift of this type, downtime during a treatment session is reduced, thereby reducing the overall time required to execute a treatment session. This in turn improves the patient's experience by reducing the amount of time they must be positioned on the patient support surface. In addition, by accommodating for movement of the target region using the primary treatment sub-plans and the secondary treatment sub-plans, it's possible to accommodate for a patient's breathing or other movement, thereby enabling a less restrictive experience for the patient. For example, the primary treatment sub-plans can accommodate a patient's breathing meaning a patient may not be required to hold their breath during a treatment fraction. Similarly, the secondary treatment sub-plans can accommodate a patient coughing or other sudden movements.

In general, the motion path 230 of the target region 200 may be three-dimensional and as such the movement boundary volume encapsulating the motion path and plurality of discrete positions *P*₁ ... *P*₇ is generally a closed surface. Figure 3a illustrates a regular grid within the movement boundary volume which indicates the positions of the plurality of discrete locations for which the secondary treatment sub-plans may be determined. In particular, the plurality of locations is indicated by the intersections of the grid lines (e.g., locations 301, 302, 303, 304, 307, 308, 309, 310 indicated by dashed circles in Figure 3a). In other words, the plurality of locations may be defined according to a regular cartesian coordinate system (e.g., the same coordinate system as used to define the discrete positions *P*₁ ... *P*₇ of the target region), which is represented by the grid in Figure 3a. As described in more detail below with reference to Figure 5, a secondary treatment sub-plan may be determined for one or more identified locations within the movement boundary volume 300. For example, a respective secondary sub-plan may be determined for each location indicated by a dashed circle shown in Figure 3a (301, 302, 303, 304, 307, 308, 309, 310), as well as every other location indicated by the intersection of the grid lines that lies within the volume 300. Alternatively, a respective secondary sub-plan may be determined for a sub-set of locations identified within the movement boundary volume 300.

In some examples, the density of locations (i.e., grid intersections) may vary throughout the movement boundary volume. The density of locations can be represented by the spacing of the grid. A small grid spacing means a higher density of locations (gridline intersections) for which secondary treatment sub-plans are determined. In contrast, a larger grid spacing means a lower density of locations for which secondary treatment sub-plans are determined. In example, the gridline spacing may be 1mm, 2mm, 5mm, or 10 mm, meaning that secondary treatment sub-plans are determined for locations within the movement boundary volume that are spaced apart by 1mm, 2mm, 5mm or 10mm in x, y, and z respectively. For example, the distance in the z axis between locations 307 and 308 as shown in Figure 3a may be 5mm. The distance locations 307 and 310 is 5mm in the x axis and 5mm in the z axis.

The density of locations within the movement boundary volume (i.e., the resolution of the movement boundary volume) therefore gives rise to a trade-off between computational resource requirements (for generating and storing the secondary treatment sub-plans for each location) and the precision afforded by a higher resolution. In other words, a higher density of locations results in a larger number of secondary sub-plans being generated for locations that are closer together (e.g., the locations may only be spaced apart by 1mm). This may result in more accurate treatment delivery, since the treatment delivery plan can be selected with high (1mm) precision. In contrast, a lower density of locations results in fewer secondary sub-plans since the locations for which the subplans are generated are spaced further apart (e.g., 10mm). This in turn results in resource savings (since less computational resources are required to generate the smaller number of secondary subplans) but may also be less accurate, since the treatment delivery plan is selected with comparatively lower (10mm) precision.

In general, the spacing between locations (and thus the precision of the treatment delivery sub-plan) may be 1mm, 2mm, 5mm, 10mm, or any value in between. In some examples, the spacing may be smaller (i.e. higher resolution) in the region of the movement boundary volume that is close to the motion path 230, and larger (i.e. lower resolution) in the region of the movement boundary volume that is further away from the motion path 230 (i.e. close to the edge of the movement boundary volume). For example, the spacing may be 1mm in the region close to the motion path, and 2mm, 5mm or 10mm in the region further away from the motion path. In other words, there may be a higher density of locations closer to the expected position (along the motion path 230) of the target region, and a comparatively lower density of locations further from the motion path. Thus, there may be a higher density of secondary treatment sub-plans in a region of the movement boundary volume closest to the motion path 230. In other words, computational resources may be focused on producing a higher density of secondary sub-plans in the region of the movement boundary volume 300 where the target region 200 is most likely to be located (in the event that the target region moves away from the expected motion path 230).

In some examples, a secondary treatment sub-plan may be generated for a sub-set of all of the locations indicated grid points within the movement boundary volume 300. For example, secondary treatment sub-plans may only be generated for locations that lie on grid points closest to the expected motion path. For example, referring to locations 307, 308 and 309 as shown in Figure 3a, a secondary treatment sub-plan may be determined for each of locations 307 and 309, but not for location 308. In other words, in some embodiments, secondary treatment sub-plans may be determined for locations defined by regular cartesian coordinates that are nearest neighbours to the plurality of discrete positions (e.g., *P*₁ ... *P*₇) or to the motion path 230. It would be appreciated that other similar techniques may be used to select a subset of locations within the movement boundary volume for which secondary sub-plans are determined. Selecting a subset in this manner reduces the overall computational cost by optimising available resources to focus on those locations that are most likely to be relevant (i.e., those locations closest to the expected motion path 230).

Referring again to Figure 3a, it can be seen that the shape and size of the movement boundary volume corresponds at least in part to the shape and size of the path defined by the plurality of discrete positions *P*₁ ... *P*₇. In general, the shape and size of the movement boundary volume (MBV) may be dictated by a number of factors and may also be determined at least in part by a clinician. The size of the movement boundary volume determines the amount of additional space (and therefore the possible number of additional discrete locations) in which it is still possible to deliver therapeutic radiation to the target region according to a secondary treatment sub-plan. Therefore, the shape and size may be determined based on a number of factors, including but not limited to the shape and/or size and/or location of the target region, and/or the proximity of the target region to heathy tissue in general or in particular adjacent organs (OARs), such as OAR 350. In general, a close proximity of the target region to a healthy organ may require a relatively smaller MBV compared to a target region that is not in close proximity to healthy organs. A clinician may determine the shape and/or size of the MBV based on the proximity of healthy tissue or nearby OARs.

In one example, the size of the movement boundary volume may be defined based on series of locations that are a set distance from the expected motion path (e.g., up to 5mm, 10mm, or 20mm in any direction from the motion path 230). The size and shape of the movement boundary volume may also be dictated by neighbouring healthy tissue or organs at risk. Referring back to Figure 3a, an organ (e.g., OAR) 350 is shown in close proximity to the expected motion path 230 of the centre 220 of the target region 200. In such examples where an OAR is close to the target region, the movement boundary volume 300 may be shaped to follow the contour of the OAR, with a sufficient "buffer" volume (the space between the boundary of the MBV 300 and the OAR 350) to avoid therapeutic radiation being applied to the healthy OAR. The shape and size of the OAR/buffer volume may be determined by a clinician depending on a number of factors, such as the shape and size of the target region, shape and size of the OAR, and precision of the treatment beam. The shape of the OAR/buffer volume is determined such that if the target region moves too close to the OAR, the treatment beam is gated to avoid any radiation being unintentionally applied to the OAR.

The movement boundary volume 300 shape and size may also be dictated by other surrounding healthy tissue that should not be subject to therapeutic radiation. In particular, the shape of the movement boundary volume 300 may follow the contour of the shape of surrounding healthy tissue. The size of the movement boundary volume may also be dictated by the proximity of surrounding tissue, the shape and size of the treatment beam, and/or any other suitable factors.

### Treatment planning

As described above, a target region 200 may move in an expected manner (e.g., according to a patient's breathing). The motion of the target region can be tracked and segmented to identify a plurality of discrete positions of the target region in a movement cycle. For each of those positions, a corresponding primary treatment sub-plan can be produced based on the target region being located at the respective position. The primary treatment sub-plan includes a large number of radiation delivery parameters that may be determined and approved by a clinician. Such parameters are described below with respect to Figure 4. The number of discrete positions of the target region for which primary treatment sub-plans are produced may vary according to requirements. A larger number of discrete positions leads to higher precision in the treatment sub-plans at the expense of increased computational resources required to generate the corresponding number of primary subplans. In contrast, a smaller number of discrete positions has a smaller computational cost since fewer primary sub-plans are produced. However, identifying a smaller number of discrete positions and producing a corresponding smaller number of primary treatment sub-plans results in lower precision in the treatment sub-plans.

In addition to the identified and expected movement, a target region may also move in an unexpected manner due to a patient coughing, sneezing, or due to another unexpected movement. A movement boundary volume is defined which contains the expected motion path of the target region (i.e., it contains all of the identified discrete positions of the target region) in addition to a plurality of other discrete locations near to but not coinciding with the discrete positions of the target region identified through imaging/tracking. A secondary treatment sub-plan can be produced for some or all of the discrete locations of the movement boundary volume such that if the target region unexpectedly moves away from the expected path, a secondary treatment plan can be selected according to the location of the target region within the movement boundary volume. This means that therapeutic radiation can continue to be delivered to the target region. The secondary treatment sub-plan includes a large number of radiation delivery parameters that may be determined and approved by a clinician. Such parameters are described below with respect to Figure 5. The number of discrete locations within the movement boundary volume for which secondary treatment sub-plans are produced may vary according to requirements. A larger number of discrete locations leads to higher precision in the treatment sub-plans at the expense of increased computational resources required to generate the corresponding number of secondary sub-plans. In contrast, a smaller number of discrete locations has a smaller computational cost due to fewer secondary sub-plans being produced. However, a smaller number of discrete locations and producing a corresponding smaller number of secondary treatment sub-plans results in lower precision in the treatment if the target region moves away from its expected motion path.

If the target region moves away from its expected path and outside of the movement boundary volume, the treatment may be stopped (i.e., the treatment beam is gated) to prevent damage to healthy tissue or nearby organs at risk.

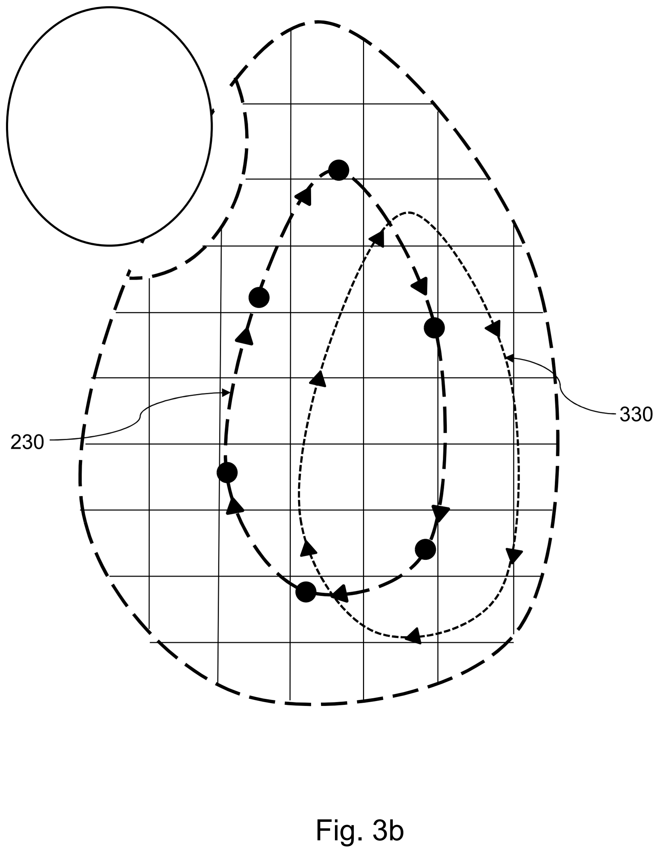

It is also envisaged that in some scenarios, a target region may systematically (i.e., permanently or semi-permanently) shift away from its expected motion path due to internal or external changes to the patient. Such a shift may result in a permanent but substantially consistent displacement of the target region from its expected path, meaning that the target region follows a new "shifted" motion path that is substantially the same as its original expected motion path, albeit that each point on the shifted path has the substantially the same displacement (in magnitude and direction) from the corresponding point on the original expected path. An example of a shifted path is indicated in Figure 3b which shows original motion path indicated by the dashed line 230 (the same as that shown in Figures 2 and 3a) as well as the new shifted motion path indicated by the dashed line 330. The shifted path 330 may have substantially the same shape and size as the original path 230 but may have a consistent relative displacement with respect to the original path as seen in Figure 3b.

In such situations, if it is determined using imaging or tracking techniques that the target region has systematically shifted away from its expected path in this manner, radiotherapy treatment can still be delivered to the target region according to some of the plurality of secondary treatment plans for some of the plurality of other discrete locations within the movement boundary volume that may lie on or near to the new shifted path. In other words, a one or more secondary treatment sub-plans can be selected according to one or more new expected positions of the target region according to the new, shifted path. The one or more secondary treatment sub plans that are selected according to the shifted path of movement for the target region may be identified at the time at which the target region is determined to have systematically deviated from its original expected path. For example, if during real-time treatment, the target region is determined (using imaging or tracking techniques) to systematically shifted to a new motion path, one or more new discrete positions of the target region may be identified from imaging or tracking data, where those new discrete positions correspond to the new shifted path, and one or more secondary treatment subplans may be selected based on the newly identified discrete positions. A radiotherapy treatment session may then be able to proceed according to the selected secondary treatment subplans that correspond to the newly identified positions of the target region.

Figures 2 and 3a above have been described in relation to the movement of a target region 200, which may be a tumour that is the target of therapeutic radiation. However, it would be appreciated that the target region may be healthy tissue, such as an organ at risk (OAR), that must be monitored to ensure it is not subject to therapeutic radiation. It would be appreciated that the techniques described above and below would apply in a similar manner to a tumour or an OAR, since radiotherapy treatment plans may still be determined for each of a plurality of positions (discrete positions identified in imaging/tracking data, as well as other possible positions outside of those identified in the imaging/tracking data) of an OAR to ensure that said treatment plans to do cause radiation to be delivered to the OAR. In general, the techniques described herein may also be used to monitor to motion of multiple target regions simultaneously, such as a first target region being a tumour and a second target region being an OAR. Primary and secondary treatment sub-plans may be determined for each of a plurality of identified/possible positions of both the tumour and the OAR, to ensure that radiation delivery is targeted to the tumour and to minimise the dose delivered to the OAR.

### Pre-treatment - Primary sub-plan generation

Figure 4 depicts a method 400 that may be performed as part of radiotherapy treatment planning (i.e., taking place at some time, such as a few days, weeks, or months before treatment) according to methods of the present disclosure.

At block 402, imaging or tracking data is received, for example from an imaging device (e.g., MRI, CT or other imaging device), from a surface-guided system such as a time-of-flight camera, or from a computer-readable memory. In the context of internal imaging, the received image data depicts the movement of a target region (e.g., target region 200), such as a tumour, of a patient over time (e.g., over time period from *t*₁ to *t*₇). In the context of tracking the target region using surface guided techniques, the received tracking data can be processed using known techniques to identify how the target region movements over the time period from *t*₁ to *t*₇. For example, the received data may indicate movement of the target region throughout multiple cycles of the patient's respiratory cycle. The received data therefore enables 3D position information (e.g., in cartesian coordinates, or another 3D coordinate system) that is correlated with time (i.e., the 3D position of the target region as the target region cyclically moves over a period of time) to be identified for the target region.

The received data may take the form of a SGRT (such as time-of-flight), CBCT, PET, 4D CT scan, an ultrasound time series, or an MR scan. In general, any form of imaging or surface tracking modality can be used that is capable of either internally imaging a target region, or externally tracking a patient's surface in order to infer internal movement of the target region, on or inside a patient's body over a period of time. For example, it is also possible to track a patient's breathing cycle respiratory tracking devices such as a breathing belt. This may be used in combination with other imaging techniques to identity the varying position of a target region over a period of time. In an example, the patient is asked to lay on the table of an imaging device, and their breathing is trained in the same way it would be trained if they were about to undergo radiotherapy treatment. The aim is to ensure the patient's breathing cycle is slow, and regular. Imaging is carried out on the target region of the patient, for example a 4DCT scan is taken. The 4DCT scan is akin to a video and allows a clinician to evaluate the movement of the patient's anatomy over the time the scan was recorded. In other examples, a patient's breathing may be tracked using a time-of-flight camera or other 3D scanning system configured to monitor the patient's surface, which may be used alone or on combination with other imaging techniques to determine how the position of the target region varies.

At step 404, a plurality of discrete positions (e.g., *P*₁ ... *P*₇) of the centre of mass of the target region are identified from the data received at step 402. In some examples, the data comprises a plurality of frames, each corresponding to a different point in time *t* over a period of time *T* in which the data was captured. Each frame of the data represents a unique position *P* of the target region at the corresponding point in time *t*. As such, it is possible to segment the data using conventional techniques known to the skilled person to identify a plurality of discrete positions (e.g., *P*₁ ... *P*₇) of the centre of mass of the target region at corresponding times *t*₁ ... *t*₇. Each position P may be provided as a set of cartesian coordinates, e.g. (*x*₁,*y*₁,*z*₁), (*x*₂,*y*_{2,}*z*₂)... (*x*₇,*y*₇,*z*₇), using a coordinate system fixed to the radiotherapy apparatus. Alternatively, any other suitable coordinate system and fixed reference frame may be used. The identified positions may be stored in computer-readable memory. The identified positions may be stored in a look-up table correlating the position of the target region to a corresponding primary treatment sub-plan as described below with reference to step 406.

In some embodiments, in the context of receiving imaging data at step 402, step 404 involves identifying the position of the centre of mass of the target region in each frame of the received image data, resulting in a large number of identified positions of the target region. As an example, the image data may comprise about 50 or more frames, optionally 100 frames or more. In one particular example, the image data is obtained using a CT scanner operating at a rate of 12 frames per second. The time period for one whole respiratory cycle (assuming a respiratory rate of about 15 breaths per second) is around 4 seconds, meaning that the image data may comprise (12 frames per second multiplied by 4 seconds) 48 frames in total. Each frame of the 48 frames represents a different discrete position of the target region as it moves during the patient's respiratory cycle. As described below with reference to step 406, a primary sub-plan is determined for each identified discrete position of the target region. Thus, embodiments that use every frame of the image data to identify the discrete positions of the target region may be desirable for producing high precision radiotherapy treatment plans, since there is a correspondingly large number of primary treatment sub-plans to select from depending on the exact position of the target region at any given time during the patient's breathing cycle.

However, the change in position of the target region between adjacent frames in the image data may be very small due to high frame rate and comparatively low movement speed of the target region. For example, the centre of mass 220 of the target region 200 may move less than 1mm in the time elapsed between adjacent frames of the image data. Movement of the target region below a certain threshold amount may not be enough to necessitate use of a different treatment sub-plan, since a sub-plan that corresponds to one position of the target region represented in one frame may be sufficiently accurate (i.e. it meets clinical objectives) for the position of the target region in an subsequent frame. As such, in some embodiments it may not be necessary to identify the discrete position of the target region in every frame of the image data. Therefore, in some embodiments, a subset of frames of the image data may be selected for identifying the discrete positions of the target region for which primary treatment sub-plans are produced.

The subset of frames may be selected based on a fixed interval between selected frames, the fixed interval being a fixed number of frames (e.g. identify the position of the target region for every Nth frame, where N is an integer, such as 2, 3, 4, 5, 6, 10 or any suitable number). Alternatively, the subset of frames may be determined based on a fixed time interval (e.g., identify the position of the target region for every frame after every M second, where M is any suitable length of time, such as 0.1 seconds, 0.5 seconds, 1 second or more). It would be appreciated that for image data acquired at a frame rate of 12 frames per second, selecting every 6^{th} frame is equivalent to selecting a frame every 0.5 seconds. The discrete position of the target region is then determined and stored in computer memory (e.g., as cartesian coordinates) for every selected frame in the subset of the frames of the image data.

In other embodiments, the subset of frames may be selected based on a threshold displacement of the centre of mass of a target region. In particular, the amount of displacement of the centre of mass of the target region between successive frames in the subset must be greater than or equal to a threshold amount, for example 1mm, 2mm, 5mm, 10mm or any other suitable value. In one example, the threshold displacement is 5mm. The threshold displacement may be determined by a clinician according to the desired accuracy and precision of the treatment plans. The first selected frame of the subset may be the first frame of the plurality of frames making up the image data. The next (i.e. second) selected frame of the subset is then the first subsequent frame (following the first frame) in the image data for which the position of the centre of mass of the target region is displaced by at least the threshold amount (e.g. 5mm) relative to the position of the centre of mass of the target region in the first frame. The next (i.e., third) selected frame of the subset is then first subsequent frame (following the second frame) in the image data for which the position of the centre of mass of the target region is displaced by at least the threshold amount relative to the position of the centre of mass of the target region in the second frame. This process for selecting the subset of frames is repeated for the whole set of frames making up the image data.

In general, techniques for identifying a motion path of a target region over a movement cycle such as a breathing cycle are known to the skilled person. In addition, techniques for segmenting image data to identify one or more positions of the target region over a movement cycle would be apparent to the skilled person. For example, whilst the description above is provided in the context of frames of image (e.g. CT, CBCT, MRI) data, the skilled person would appreciate that the same segmentation techniques for identifying the discrete positions of the target region can be applied to position data for the target region that has been inferred from surface-guided tracking techniques.

Turning now to step 406, a primary treatment sub-plan, such as one described above in relation to Figure 2, is determined for each discrete position of the target region identified at step 404. As a result, a plurality of primary treatment sub-plans is generated, each one associated with a corresponding discrete position of the target region within the target region's motion cycle. Each primary treatment sub-plan may be associated with the corresponding discrete position in computer memory using any suitable technique, such as a look-up table.

In more detail, the primary treatment sub-plan for any particular discrete position may be determined based on the patient's anatomy when the target region is located at the particular discrete position. For example, the treatment sub-plan for a particular discrete position may be based on the target region's position as well as one or more of its other expected characteristics at the discrete position such as the target region's shape or size. In addition, and other aspects of the patient's anatomy such as the location and shape of nearby OARs may be taken into account when preparing the treatment plan, particularly where these OARs are associated with treatment parameters prescribed by the clinician.

As an example, while the target region is located at discrete position *P*₁ (e.g. while the centroid of the target region is located at *P*₁), it has been determined (according to the pre-treatment data) that the target region has a particular expected location and a particular expected shape, and that any surrounding organs at risk each have a particular expected location and shape. Known treatment planning techniques and algorithms may be adapted in order to take this information into account, and to determine optimal radiation delivery parameters for each primary treatment sub-plan according to each identified discrete position. As an example, each primary treatment sub-plan may be determined such that the amount of radiation delivered to healthy tissue (e.g., surrounding organs at risk) is minimised.

Each primary sub-plan comprises a respective set of radiation delivery parameters defining radiation delivery to the patient, wherein the radiation delivery parameters include one or more of at least the following:
gantry rotation angle of delivery;
gantry tilt angle of delivery;
radiotherapy beam shape per rotation and/or tilt angle of delivery;
radiotherapy beam energy per rotation and/or tilt angle of delivery; and
the duration of application of the radiotherapy beam per rotation and/or tilt angle of delivery; and
patient position per gantry rotation angle.

The skilled person will appreciate that each primary treatment sub-plan may include radiation delivery from multiple beam angles (e.g., gantry rotation angle and/or non-coplanar tilt angle). For example, for a treatment fraction in which radiation is to be delivered from six angles, a single subplan may include sets of delivery parameters that define radiation delivery from each of those six angles. Each sub-plan therefore takes into account the expected location and shape of the target region, and OARs, from the beam's-eye-view at each delivery angle.

As would be appreciated by the skilled person, the set of radiation delivery parameters may further include any number of additional parameters according to which radiation is delivered to the patient. In general, the skilled person would be aware of various techniques for determining a radiotherapy treatment plan comprising a set of radiation delivery parameters.

At block 408, approval for each of the primary treatment sub-plans may optionally be sought and obtained from a clinician. In practice, this may involve the clinician manually approving the first treatment sub-plan (corresponding to the first identified discrete position of the target region).

Following manual approval of the first sub-plan, every subsequent primary treatment sub-plan that is within the same treatment objective boundaries may be automatically approved. However, if there are any sub-plans that do not fulfil the same objectives, they may need to be approved separately. For example, is a tumour in the lung moves to close to the spine during breathing, a clinician may need to separately approve any sub-plan associated with a position of the tumour that is close to the spine, to ensure that the spine does not receive a dose of radiation that exceeds a limit.

In general, the method 400 of Figure 4 allows a motion path of a target region to be identified over a breathing cycle at the time the imaging/tracking data was collected. The motion is segmented into discrete positions of the target region over the cycle, using selected frames of the data or otherwise using selected positions of the target region identified from the data. It is generally expected that a patient's breathing cycle will not significantly change during the time between the pre-treatment planning (method 400) and the time of treatment itself (method 600 described below). Thus, the recorded movement of a target region over a breathing cycle during the pre-treatment phase will be very similar to the breathing cycle during treatment.

### Pre-treatment - Secondary sub-plan generation

Figure 5 depicts a further method 500 that may be performed as part of radiotherapy treatment planning, in which secondary treatment sub-plans are be generated concurrently with, or after, the generation of the primary treatment sub-plans according to method 400. It would be appreciated that method 500 is optional, meaning that in some embodiments, radiotherapy treatment planning only involves generating one or more primary treatment sub-plans based on actual identified locations of the target region. In these embodiments, the radiotherapy treatment itself will therefore be based only on primary treatment sub-plans.

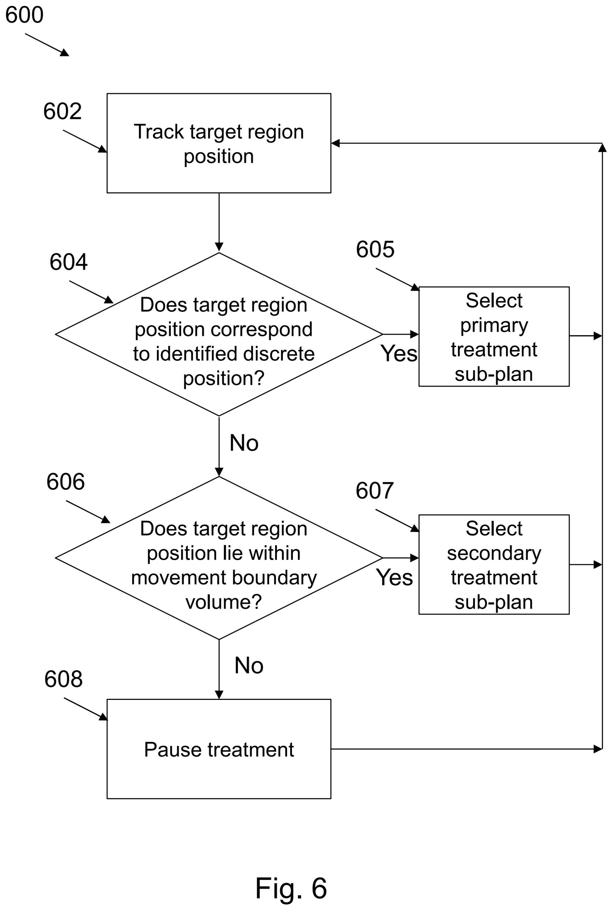

However, in other embodiments, radiotherapy treatment planning may involve determining one or more secondary treatment sub-plans according to other possible positions of the target region outside of the identified discrete positions. As such, the radiotherapy treatment itself may deliver radiation according to a combination of primary and secondary sub-plans, depending on the position(s) of the target region during the treatment. This is described in more detail below with reference to Figure 6.

Returning to Figure 5, the method 500 comprises a step 502 of determining a movement boundary volume. As described above with reference to Figure 3a, the movement boundary volume encapsulates (i.e., completely contains) each of the identified discrete positions (identified at step 404) of the target region. The movement boundary volume further comprises an additional volume of space around the target region for which secondary treatment sub-plans are determined according to the steps described below. The movement boundary volume shape and size may be determined using the same data (received at step 402) that is used to identify the discrete positions of the target region for generating the primary treatment sub-plans. In addition, the movement boundary volume shape and size may be determined by a clinician. For example, the clinician may identify organs at risk near to the target region which should not be included in the movement boundary volume. As discussed above with reference to Figure 3a, the shape and size of the movement boundary volume may be dictated by other physiological factors or by parameters associated with the radiotherapy system used to treat the patient.

Following step 502, method 500 comprises a step 504 of identifying locations within the movement boundary volume for which corresponding secondary treatment sub-plans can be produced. As described above, the locations may be defined using a coordinate system (e.g., cartesian) fixed to the radiotherapy system or another entity. As shown in Figure 3a, the locations within the movement boundary volume may be represented by a grid, where each location is identified at an intersection of x, y, and z grid lines. In general, the locations may be identified as a regular cubic array, where separated by a fixed and predefined amount in each of x, y and z. As discussed above with reference to Figure 3a, the plurality of locations may have varying density throughout the movement boundary volume. For example, there may be a higher density of locations for which secondary treatment sub-plans are produced near to the discrete positions (identified at step 404), and a relatively lower density of locations towards the edge of the movement boundary volume. In general, the density of locations within the movement boundary volume may have a fixed or varying density.

After identifying the secondary locations within the movement boundary volume, method 500 proceeds to step 506 of determining, for each location identified at step 504, a secondary treatment sub-plan. As a result, a plurality of secondary treatment sub-plans are generated, each one associated with a corresponding location in the movement boundary volume. Each secondary treatment sub-plan may be associated with the corresponding location in computer memory using any suitable technique, such as a look-up table.

In general, the process for determining each secondary treatment sub-plan for the locations within the movement boundary volume is similar or identical to the process 406 for determining each primary treatment sub-plan for the identified positions of the target region in the imaging/tracking data. In particular, the process for determining each secondary treatment sub-plan is carried out on the basis that the centre of mass (220) of the target region (200) is located at the corresponding location within the movement boundary volume. Each secondary sub-plan may be based on the target region's position at the corresponding location, as well as one or more of its other expected characteristics at the discrete position such as the target region's shape or size. In addition, and other aspects of the patient's anatomy such as the location and shape of nearby OARs may be taken into account when preparing the treatment plan, particularly where these OARs are associated with treatment parameters prescribed by the clinician. In general, known treatment planning techniques and algorithms may be adapted in order to take this information into account, and to determine optimal radiation delivery variables for each primary treatment sub-plan according to each identified discrete position. As an example, each primary treatment sub-plan may be determined such that the amount of radiation delivered to healthy tissue (e.g., surrounding organs at risk) is minimised.

Each secondary treatment sub-plan comprises a respective set of radiation delivery parameters defining radiotherapy delivery to the patient. The parameters of each secondary sub-plan may be similar to or identical those of the primary sub-plans. For example, the parameters may include:
gantry rotation angle of delivery;
gantry tilt angle of delivery;
radiotherapy beam shape per rotation and/or tilt angle of delivery;
radiotherapy beam energy per rotation and/or tilt angle of delivery; and
the duration of application of the radiotherapy beam per rotation and/or tilt angle of delivery;
patient position per gantry rotation angle;
and any other suitable parameters according to which radiation is delivered to the patient.

At block 508, approval for each of the primary treatment sub-plans may optionally be sought and obtained from a clinician in the same manner as approving each primary treatment sub-plan according to step 410. In practice, this may involve the clinician manually approving the one secondary treatment sub-plan. Following manual approval of the first secondary sub-plan, every other secondary sub-plan for the remaining locations within the movement boundary volume may be automatically approved. However, in some examples, secondary sub-plans corresponding to locations within the movement boundary volume that are near to healthy tissue or OARs (e.g., at the edge of the movement boundary volume) may each need manual review and approval by a clinician.

### Radiotherapy treatment

In general, methods 400 and 500 are performed prior to treatment, for example as part of offline treatment planning. Determining the primary and secondary sub-plans, each with their own radiation delivery variables, not only for the actual identified positions of the target region (primary) but also for locations outside of the expected positions and within the movement boundary volume (secondary), requires a large amount of processing power. This is particularly so considering that sub-plans for each target point may involve radiation delivery from each of a plurality of beam angles. However, by conducting the computation before treatment in the manner depicted in the flowcharts of Figures 4 and 5, it follows that during treatment, radiotherapy control software can react quickly to movement of the target region due to patient breathing, coughing, and so on. In particular, monitoring the location and/or shape of the tumour during treatment means that the position of the target region, and deviations in shape, can be monitored and the corresponding subplan associated with the monitored position/shape can be selected and implemented immediately, or else with significantly reduced delay due to processing time. Furthermore, because each sub-plan has received prior approval from a clinician, it can be ensured that the control software is operating within the limits of appropriate clinical oversight at all times during treatment.

Figure 6 depicts a method 600 according to the present disclosure of selecting a radiotherapy treatment plan according to the present disclosure. Optionally, the method 600 comprises configuring a radiotherapy treatment apparatus according to the selected treatment plan and or/controlling a radiation therapy delivery system according to the selected treatment plan. The radiotherapy delivery system comprises an imaging or surface-tracking apparatus, and may be as described above with respect to figure 1.

At step 602, a motion of a target region 200 is monitored during treatment using signals from the imaging/tracking apparatus. The signals may be received, for example, from an MR imaging apparatus, surface camera, CBCT imaging system, PET system, or ultrasound system. Monitoring the motion of the target region in this way during treatment can be accomplished using known techniques, including known auto-segmentation techniques. In other words, the location and optionally the shape of the target region, is tracked during treatment. In some examples, the location and optionally the shape of organs at risk near to the target region may also be monitored. Monitoring the position of a target region may involve monitoring a centre of mass of the target region as described above with respect to Figures 2 and 3.

At step 604, the method 600 involves determining whether a real-time position of the target region corresponds to one of the plurality of discrete positions identified during the planning phase as described above with respect to Figure 4. In other words, the method involves determining whether the instantaneous real-time position of the target region identified at step 602 corresponds to an expected position of the target region according to an expected motion cycle.

In more detail, step 604 involves determining whether the real-time position of the target region is at or within a threshold distance of one of the plurality of discrete positions identified during the planning phase. The threshold distance may be determined by the clinician, for example 1mm, 2mm, 3mm, 5mm, 10mm or any suitable distance. In some examples, the threshold distance may correspond to the threshold displacement between adjacent discrete positions. For example, if the identified positions are each 3mm apart, the threshold distance used in step 604 may be 3mm. This means that if the real-time position of the target region is within 3mm of one of the plurality of discrete positions, then the real-time position of the target region is considered to correspond to that discrete position for the purposes of step 604.

If at step 604, it is determined that the real-time position of the target region does correspond to one of the discrete positions, that is to say the real-time position is within a predetermined threshold distance of one of the discrete positions, the method proceeds to step 605 at which a primary treatment sub-plan is selected. Selecting a primary treatment sub-plan may involve using a look-up table that associates the identified discrete positions with their respective primary treatment sub-plans, as described above with reference to Figure 4. The primary sub-plan that is selected is associated with the discrete position that the real-time target position corresponds to. In other words, at step 605, the discrete position that is determined to be closest to the real-time position of the target region is used to select the corresponding primary treatment sub-plan.

In some examples, selecting the primary treatment sub-plan may further comprise configuring components of the radiotherapy system according to parameters of the selected treatment subplan. For example, this may involve rotating the gantry according to a rotation angle parameter of the selected primary sub-plan, configuring a tilt angle according to a tilt angle parameter of the selected primary sub-plan configuring the multi-leaf collimator according to a corresponding parameter of the selected primary sub-plan, and/or any other suitable physical configuration of the radiotherapy system based on the selected primary sub-plan. Step 605 may further comprise controlling the radiotherapy system to deliver treatment to the patient according to the selected primary sub-plan.

If at step 604, it is determined that the real-time position of the target region does not correspond to one of the discrete positions, that is to say the real-time position Is not within a predetermined threshold of one of the discrete positions identified in the motion cycle, the method proceeds to step 606. At step 606, the method involves determining whether the real-time position of the target region corresponds to one of the plurality of locations (e.g. 301, 302, 303, and so on) within the movement boundary volume 300 identified during the planning phase as described above with respect to Figure 5. In other words, the method involves determining whether the instantaneous real-time position of the target region identified at step 602, if not corresponding to an expected position along the target region motion cycle, lies within the extended volume of space provided by the movement boundary volume 300.

In more detail, step 606 involves determining whether the real-time position of the target region is at or within a threshold distance of one of the locations within the movement boundary volume identified during the planning phase. The threshold distance may be determined by the clinician, for example 1mm, 2mm, 3mm, 5mm, 10mm or any suitable distance. In some examples, the threshold distance may correspond to the spacing between adjacent identified locations. For example, if the locations are each 3mm apart, the threshold distance used in step 606 may be 3mm. This means that if the real-time position of the target region is within 3mm of one of the locations within the movement boundary volume, then the real-time position of the target region is considered to correspond to that location for the purposes of step 606.

If at step 606, it is determined that the real-time position of the target region does correspond to one of the locations within the movement boundary volume, that is to say the real-time position is within a predetermined threshold distance of one of the locations, the method proceeds to step 607 at which a secondary treatment sub-plan is selected. Selecting a secondary treatment sub-plan may involve using a look-up table that associates the identified locations with their respective secondary treatment sub-plans, as described above with reference to Figure 5. The secondary sub-plan that is selected is associated with the location that the real-time target position corresponds to. In other words, at step 607, the identified location within the movement boundary volume that is determined to be closest to the real-time position of the target region is used to select the corresponding secondary treatment sub-plan.

In analogy to step 605, selecting the secondary treatment sub-plan at step 607 may further comprise configuring components of the radiotherapy system according to parameters of the selected treatment sub-plan. Step 607 may further comprise controlling the radiotherapy system to deliver treatment to the patient according to the selected primary sub-plan.

If at step 606, it is determined that the real-time position of the target region does not correspond to one of the identified locations within the movement boundary volume, that is to say the real-time position Is not within a predetermined threshold of one of the locations in the movement boundary volume, the method proceeds to step 608 at which radiotherapy treatment is paused. This is because the real-time position of the target region is neither on an expected path of a motion cycle nor is it located within a volume which is has been determined to be safe for continuing to delivery radiotherapy treatment according to a secondary sub-plan. In other words, reaching step 608 means that the position of the target region is not associated with a predetermined treatment plan and therefore treatment must be paused.

As demonstrated by the arrows in Figure 6, method 600 is iterative and may be repeated continually throughout a treatment session. In other words, during treatment, the position of the target region is continually tracked to determine whether a current treatment plan needs to be switched to a new treatment plan as the target region moves. Under a patient's normal breathing cycle, the position of the target region will move according to the expected motion path, passing through each identified discrete position along the motion cycle. As this happens, the imaging or tracking system of the radiotherapy system can monitor the position of the target region and continually deliver treatment by updating the selection of the treatment plan using method 600. Using both primary and secondary treatment sub-plans, the radiotherapy system can continually deliver the treatment even if there is an unexpected change of position of the target region away from the expected motion path, by updating the selection of treatment sub-plan using a corresponding secondary sub-plan.

As discussed above, the method depicted in Figure 6 may also be used in the event that the target region is determined (e.g., at step 602) to have systematically shifted away from its expected path along which each originally identified discrete position lies. In this example, a plurality of new discrete positions may be identified based on imaging/tracking data, or based on calculations using a) information indicating the magnitude and direction of the displacement of the target region from it's expected path and b) the originally identified discrete positions. In particular, a single shifted position of the target region may be extrapolated, using the originally identified discrete positions of the target region, to a new set of shifted discrete positions, where each position in the new set is displaced by the same amount and in the same direction relative to its corresponding position in the original set of discrete positions. Using the new set of discrete positions, a new set of sub-plans can be selected from the plurality of secondary treatment sub-plans, where each secondary subplan that is selected corresponds to one of the newly identified discrete positions. Radiotherapy treatment can then proceed according to this set of secondary treatment sub-plans in the same manner as described above as the target region moves along the shifted motion path and through the set of new, shifted discrete positions.

### Radiotherapy System

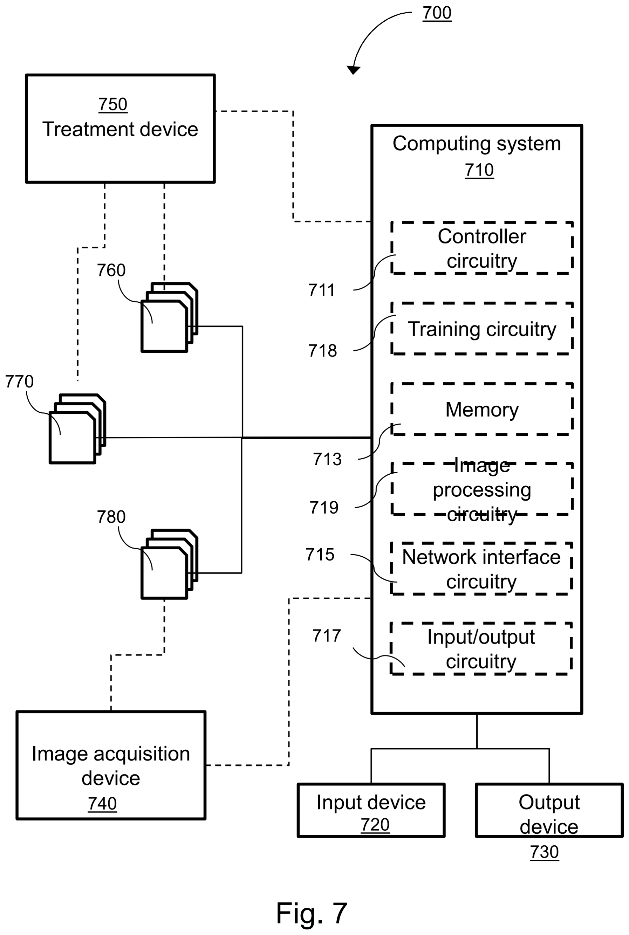

Figure 7 illustrates a block diagram of one implementation of a radiotherapy system 700. The radiotherapy system 700 comprises a computing system 710 within which a set of instructions, for causing the computing system 710 to perform any one or more of the methods discussed herein, may be executed.

The computing system 710 shall be taken to include any number or collection of machines, e.g., computing device(s), that individually or jointly execute a set (or multiple sets) of instructions to perform any one or more of the methods discussed herein. That is, hardware and/or software may be provided in a single computing device, or distributed across a plurality of computing devices in the computing system. In some implementations, one or more elements of the computing system may be connected (e.g., networked) to other machines, for example in a Local Area Network (LAN), an intranet, an extranet, or the Internet. One or more elements of the computing system may operate in the capacity of a server or a client machine in a client-server network environment, or as a peer machine in a peer-to-peer (or distributed) network environment. One or more elements of the computing system may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine.

The computing system 710 includes controller circuitry 711 and a memory 713 (e.g., read-only memory (ROM), flash memory, dynamic random-access memory (DRAM) such as synchronous DRAM (SDRAM) or Rambus DRAM (RDRAM), etc.). The memory 713 may comprise a static memory (e.g., flash memory, static random-access memory (SRAM), etc.), and/or a secondary memory (e.g., a data storage device), which communicate with each other via a bus (not shown).

Controller circuitry 711 represents one or more general-purpose processors such as a microprocessor, central processing unit, accelerated processing units, or the like. More particularly, the controller circuitry 711 may comprise a complex instruction set computing (CISC) microprocessor, reduced instruction set computing (RISC) microprocessor, very long instruction word (VLIW) microprocessor, processor implementing other instruction sets, or processors implementing a combination of instruction sets. Controller circuitry 711 may also include one or more special-purpose processing devices such as an application specific integrated circuit (ASIC), a field programmable gate array (FPGA), a digital signal processor (DSP), network processor, or the like. One or more processors of the controller circuitry may have a multicore design. Controller circuitry 711 is configured to execute the processing logic for performing the operations and steps discussed herein.

The computing system 710 may further include a network interface circuitry 715. The computing system 710 may be communicatively coupled to an input device 720 and/or an output device 730, via input/output circuitry 717. In some implementations, the input device 720 and/or the output device 730 may be elements of the computing system 710. The input device 720 may include an alphanumeric input device (e.g., a keyboard or touchscreen), a cursor control device (e.g., a mouse or touchscreen), an audio device such as a microphone, and/or a haptic input device. The output device 730 may include an audio device such as a speaker, a video display unit (e.g., a liquid crystal display (LCD) or a cathode ray tube (CRT)), and/or a haptic output device. In some implementations, the input device 720 and the output device 730 may be provided as a single device, or as separate devices.

In some implementations, the computing system 710 may comprise image processing circuitry 719. Image processing circuitry 719 may be configured to process image data 780 (e.g., images, or imaging data), such as medical images obtained from one or more imaging data sources, a treatment device 750 and/or an image acquisition device 740. Image processing circuitry 719 may be configured to process, or pre-process, image data. For example, image processing circuitry 719 may convert received image data into a particular format, size, resolution or the like. In some implementations, image processing circuitry 719 may be combined with controller circuitry 711.

In some implementations, the radiotherapy system 700 may further comprise an image acquisition device 740 and/or a treatment device 750, such as those disclosed herein in the examples of Figure 1. The image acquisition device 740 and the treatment device 750 may be provided as a single device. In some implementations, treatment device 750 is configured to perform imaging, for example in addition to providing treatment and/or during treatment. The treatment device 750 comprises the main radiation delivery components of the radiotherapy system, such as the Linac, beam guides and beam shaping systems including a multi-leaf collimator.

Image acquisition device 740 may be configured to perform positron emission tomography (PET), computed tomography (CT), magnetic resonance imaging (MRI), or any other suitable imaging technique. Image acquisition device 740 may be configured to output image data 780, which may be accessed by computing system 710. Treatment device 750 may be configured to output treatment data 760, which may be accessed by computing system 710.

Computing system 710 may be configured to access or obtain treatment data 760, planning data 770 and/or image data 780. Treatment data 760 may be obtained from an internal data source (e.g., from memory 713) or from an external data source, such as treatment device 750 or an external database. Planning data 770 may be obtained from memory 713 and/or from an external source, such as a planning database. Planning data 770 may comprise information obtained from one or more of the image acquisition device 740 and the treatment device 750.

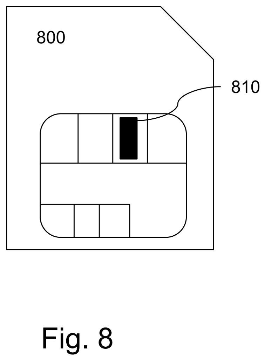

The various methods described above may be implemented by a computer program. The computer program may include computer code (e.g., instructions) 810 arranged to instruct a computer to perform the functions of one or more of the various methods described above. The steps of the methods described above may be performed in any suitable order. For example, step 604 of method 600 may be performed simultaneously or substantially simultaneously with step 606. The computer program and/or the code 810 for performing such methods may be provided to an apparatus, such as a computer, on one or more computer readable media or, more generally, a computer program product 800)), depicted in Figure 8. The computer readable media may be transitory or non-transitory. The one or more computer readable media 800 could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid-state memory, magnetic tape, a removable computer diskette, a random-access memory (RAM), a read-only memory (ROM), a rigid magnetic disc, and an optical disk, such as a CD-ROM, CD-R/W or DVD. The instructions YY10 may also reside, completely or at least partially, within the memory 713 and/or within the controller circuitry 711 during execution thereof by the computing system 710, the memory 713 and the controller circuitry 711 also constituting computer-readable storage media.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as " receiving", "determining", "comparing ", "enabling", "maintaining," "identifying", or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

The following embodiments are disclosed:
1. A computer-implemented method for determining a radiotherapy treatment plan to be carried out using a radiotherapy device comprising a radiotherapy beam source mounted to a gantry, the radiotherapy beam source configured to irradiate a subject with a radiotherapy beam, wherein the method comprises:
   receiving data indicating movement of a target region of a patient over a period of time;
   identifying, based on the received data, a plurality of discrete positions of the target region, each discrete position of the plurality of positions corresponding to a different time within the period of time; and
   determining, for each of the plurality of discrete positions, a respective primary treatment sub-plan;
      wherein each primary treatment sub-plan comprises a respective set of radiation delivery parameters defining radiotherapy delivery to the patient, wherein the radiation delivery parameters include one or more of:
   gantry rotation angle of delivery;
   gantry tilt angle of delivery;
   radiotherapy beam shape per rotation angle and/or tile angle of delivery;
   radiotherapy beam energy per rotation angle and/or tilt angle of delivery;
   the duration of application of the radiotherapy beam per rotation angle and/or tilt angle of delivery; and
   patient position per gantry rotation angle and/or tilt angle of delivery; and
   wherein the set of radiation delivery parameters is determined for each primary treatment sub-plan based on the respective discrete position of the target region.
2. The method of clause 1, further comprising creating a primary look-up table that associates one or more locations of the target region over the period of time with the respective primary treatment sub-plan.
3. The method of clause 1 or 2, wherein the plurality of discrete positions of the target region are based on a location of centre of mass of the target region.
4. The method of any preceding clause, wherein the target region comprises healthy tissue.
5. The method of any preceding clause, wherein the set of radiation delivery parameters determined for each primary treatment sub-plan is further based on a shape of the target region at the respective discrete position.
6. The method of any preceding clause, further comprising:
   determining a movement boundary volume around the target region, wherein the movement boundary volume is a volume of space encapsulating each of the plurality of discrete positions of the target region;
   identifying a plurality of locations within the movement boundary volume that do not correspond to any of the plurality of discrete positions of the target region; and
   determining, for each identified location, a respective secondary treatment subplan;
   wherein each secondary treatment sub-plan comprises a respective set of radiation delivery parameters defining radiotherapy delivery to the patient, and wherein the set of radiation delivery parameters is determined for each secondary sub-plan based on centre of mass of the target region being located at the corresponding location within the movement boundary volume.
7. The method of clause 6, further comprising creating a secondary look-up table that associates the one or more locations within the movement boundary volume that do not correspond to the discrete positions of the target region with the respective secondary treatment sub-plan.
8. The method of clause 6 or 7, wherein the size of the boundary movement volume is determined by one or more of:
   a size of the target region;
   a shape of the target region;
   a type of healthy tissue adjacent to the target region;
   a proximity of the target region to adjacent healthy tissue; and
   a shape or size of the radiotherapy beam.
9. The method of any preceding clause, wherein the movement of the target region over the period of time defines a motion path and wherein each primary sub-plan corresponds to a different position along the motion path.
10. The method of clause 9 when dependent on any of clauses 6 to 8, wherein each secondary sub-plan corresponds to a location within the movement boundary volume that is not on the motion path.
11. The method of any preceding clause, wherein the received data comprises a plurality of image frames, wherein each frame identifies one of the discrete positions of the plurality of discrete positions of the target region, and wherein each primary sub-plan corresponds to the discrete position of target region in a respective one of the plurality of frames.
12. The method of clause 11, wherein determining the primary treatment sub-plans comprises determining a primary treatment sub-plan for every Nth frame of the plurality of frames, where N is a predetermined frame interval number.
13. The method of clause 11, wherein determining the plurality of primary treatment sub-plans comprises determining a primary treatment sub-plan for each frame of a subset of the plurality of frames, wherein the subset of frames are selected such that a displacement of the position of the centre of mass of the target region between successive frames in the subset is greater than or equal to a threshold displacement.
14. The method of clause 13, wherein the threshold displacement amount is 10mm or less, optionally 5mm or less, optionally 2mm or less, optionally 1mm.
15. The method of clause 13 or 14, wherein the threshold displacement represents an amount of movement of the target region away from any discrete position of the target region for which it is acceptable to provide radiotherapy treatment according to a primary treatment sub-plan that corresponds to the given discrete position.
16. The method of clause 15, wherein the amount of movement of the target region is determined by one or more factors, including one or more of: a size of the target region, a shape of the target region, a type of healthy tissue adjacent to the target region, a proximity of the target region to the adjacent healthy tissue, a shape or size of the radiotherapy beam.
17. A computer-implemented method for determining a radiotherapy treatment plan to be carried out using a radiotherapy device, wherein the radiotherapy device comprises a radiotherapy beam source mounted to a gantry, the radiotherapy beam source configured to irradiate a subject with a radiotherapy beam, wherein the method comprises:
   receiving data indicating position information for a target region of a patient;
   determining, based on the received data, a current position of the target region; and
   selecting, based on the current position of the target region, a treatment sub-plan from a plurality of predetermined treatment sub plans, wherein each of the predetermined treatment sub-plans corresponds to a different position of the target region and wherein the selected treatment sub-plan corresponds to the determined current position of the target region;
   wherein each sub-plan comprises a respective set of radiation delivery parameters defining radiotherapy delivery to the patient, wherein the radiation delivery parameters include one or more of:
      gantry rotation angle of delivery;
      gantry tilt angle of delivery;
      radiotherapy beam shape per rotation angle and/or tile angle of delivery;
      radiotherapy beam energy per rotation angle and/or tilt angle of delivery;
      the duration of application of the radiotherapy beam per rotation angle and/or tilt angle of delivery; and
      patient position per gantry rotation angle and/or tilt angle of delivery.
18. The method of clause 17, further comprising sending a control signal to the radiotherapy device based on the selected sub-plan to cause the radiotherapy device to deliver radiotherapy to the patient in accordance with the set of radiation delivery parameters of the selected treatment sub-plan.
19. The method of clause 17 or 18, wherein selecting the treatment sub-plan comprises:
   accessing one or more look-up tables that store an association between target region positions and corresponding predetermined treatment sub-plans that were determined according to the respective target region position; and
   selecting a predetermined treatment sub-plan from the look-up table based on the associated target region position that matches the determined target region position.
20. The method of any of clause 17 to 19, further comprising:
   receiving updated data indicating position information for the target region of the patient;
   determining, based on the updated data, an updated position of the target region; and
   selecting, based on the updated position of the target region, a second treatment sub-plan from the plurality of predetermined treatment sub-plans that corresponds to the updated position of the target region.
21. The method of clause 19, further comprising sending an updated control signal to the radiotherapy device based on the selected second sub-plan to cause the radiotherapy device to deliver radiotherapy to the patient in accordance with the radiation delivery parameters of the second sub-plan.
22. The method of any of clause 17 to 21, wherein selecting a treatment sub-plan from a plurality of predetermined treatment sub plans comprises:
   determining whether the current position of the target region corresponds to a predetermined position of the target region identified during a treatment planning phase;
   responsive to determining that the current position of the target region corresponds to any one of a plurality of predetermined positions of the target region identified during a treatment planning phase:
      selecting a treatment sub-plan from a first set of treatment sub-plans, the first set of treatment sub-plans each corresponding to a different predetermined position of the target region, wherein the selected treatment sub-plan corresponds to the current position of the target region;
   responsive to determining that the current position of the target region does not correspond to one of the plurality of predetermined position of the target region:
      determining that the current position of the region corresponds to a location within a predetermined volume of space encapsulating the plurality of predetermined positions; and
      selecting a treatment sub-plan from a second set of treatment sub-plans, the second set of treatment sub-plans each corresponding to a different location within the predetermined volume of space, wherein the selected treatment subplan corresponds to the current position of the target region.

## Claims

1. A computer-implemented method for determining a radiotherapy treatment plan to be carried out using a radiotherapy device comprising a radiotherapy beam source mounted to a gantry, the radiotherapy beam source configured to irradiate a subject with a radiotherapy beam, wherein the method comprises:
receiving data indicating movement of a target region of a patient over a period of time;
identifying, based on the received data, a plurality of discrete positions of the target region, each discrete position of the plurality of positions corresponding to a different time within the period of time; and
determining, for each of the plurality of discrete positions, a respective primary treatment sub-plan;
wherein each primary treatment sub-plan comprises a respective set of radiation delivery parameters defining radiotherapy delivery to the patient, wherein the radiation delivery parameters include one or more of:
gantry rotation angle of delivery;
gantry tilt angle of delivery;
radiotherapy beam shape per rotation angle and/or tile angle of delivery;
radiotherapy beam energy per rotation angle and/or tilt angle of delivery;
the duration of application of the radiotherapy beam per rotation angle and/or tilt angle of delivery; and
patient position per gantry rotation angle and/or tilt angle of delivery; and
wherein the set of radiation delivery parameters is determined for each primary treatment sub-plan based on the respective discrete position of the target region.

2. The method of claim 1, further comprising creating a primary look-up table that associates one or more locations of the target region over the period of time with the respective primary treatment sub-plan.

3. The method of claim 1 or 2, wherein the plurality of discrete positions of the target region are based on a location of centre of mass of the target region.

4. The method of any preceding claim, wherein the target region comprises healthy tissue.

5. The method of any preceding claim, wherein the set of radiation delivery parameters determined for each primary treatment sub-plan is further based on a shape of the target region at the respective discrete position.

6. The method of any preceding claim, further comprising:
determining a movement boundary volume around the target region, wherein the movement boundary volume is a volume of space encapsulating each of the plurality of discrete positions of the target region;
identifying a plurality of locations within the movement boundary volume that do not correspond to any of the plurality of discrete positions of the target region; and
determining, for each identified location, a respective secondary treatment subplan;
wherein each secondary treatment sub-plan comprises a respective set of radiation delivery parameters defining radiotherapy delivery to the patient, and wherein the set of radiation delivery parameters is determined for each secondary sub-plan based on centre of mass of the target region being located at the corresponding location within the movement boundary volume, optionally further comprising creating a secondary look-up table that associates the one or more locations within the movement boundary volume that do not correspond to the discrete positions of the target region with the respective secondary treatment sub-plan.

7. The method of claim 6, wherein the size of the boundary movement volume is determined by one or more of:
a size of the target region;
a shape of the target region;
a type of healthy tissue adjacent to the target region;
a proximity of the target region to adjacent healthy tissue; and
a shape or size of the radiotherapy beam.

8. The method of any preceding claim, wherein the movement of the target region over the period of time defines a motion path and wherein each primary sub-plan corresponds to a different position along the motion path.

9. The method of claim 8 when dependent on claim 6 or 7, wherein each secondary sub-plan corresponds to a location within the movement boundary volume that is not on the motion path.

10. The method of any preceding claim, wherein the received data comprises a plurality of image frames, wherein each frame identifies one of the discrete positions of the plurality of discrete positions of the target region, and wherein each primary sub-plan corresponds to the discrete position of target region in a respective one of the plurality of frames, optionally wherein determining the primary treatment sub-plans comprises determining a primary treatment sub-plan for every Nth frame of the plurality of frames, where N is a predetermined frame interval number, and optionally wherein determining the plurality of primary treatment sub-plans comprises determining a primary treatment sub-plan for each frame of a subset of the plurality of frames, wherein the subset of frames are selected such that a displacement of the position of the centre of mass of the target region between successive frames in the subset is greater than or equal to a threshold displacement.

11. The method of claim 10, wherein the threshold displacement represents an amount of movement of the target region away from any discrete position of the target region for which it is acceptable to provide radiotherapy treatment according to a primary treatment sub-plan that corresponds to the given discrete position, optionally wherein the amount of movement of the target region is determined by one or more factors, including one or more of: a size of the target region, a shape of the target region, a type of healthy tissue adjacent to the target region, a proximity of the target region to the adjacent healthy tissue, a shape or size of the radiotherapy beam.

12. A computer-implemented method for determining a radiotherapy treatment plan to be carried out using a radiotherapy device, wherein the radiotherapy device comprises a radiotherapy beam source mounted to a gantry, the radiotherapy beam source configured to irradiate a subject with a radiotherapy beam, wherein the method comprises:
receiving data indicating position information for a target region of a patient;
determining, based on the received data, a current position of the target region; and
selecting, based on the current position of the target region, a treatment sub-plan from a plurality of predetermined treatment sub plans, wherein each of the predetermined treatment sub-plans corresponds to a different position of the target region and wherein the selected treatment sub-plan corresponds to the determined current position of the target region;
wherein each sub-plan comprises a respective set of radiation delivery parameters defining radiotherapy delivery to the patient, wherein the radiation delivery parameters include one or more of:
gantry rotation angle of delivery;
gantry tilt angle of delivery;
radiotherapy beam shape per rotation angle and/or tile angle of delivery;
radiotherapy beam energy per rotation angle and/or tilt angle of delivery;
the duration of application of the radiotherapy beam per rotation angle and/or tilt angle of delivery; and
patient position per gantry rotation angle and/or tilt angle of delivery.

13. The method of claim 12, wherein selecting the treatment sub-plan comprises:
accessing one or more look-up tables that store an association between target region positions and corresponding predetermined treatment sub-plans that were determined according to the respective target region position; and
selecting a predetermined treatment sub-plan from the look-up table based on the associated target region position that matches the determined target region position.

14. The method of claim 13, further comprising:
receiving updated data indicating position information for the target region of the patient;
determining, based on the updated data, an updated position of the target region; and
selecting, based on the updated position of the target region, a second treatment sub-plan from the plurality of predetermined treatment sub-plans that corresponds to the updated position of the target region.

15. The method of any of claims 12 to 14, wherein selecting a treatment sub-plan from a plurality of predetermined treatment sub plans comprises:
determining whether the current position of the target region corresponds to a predetermined position of the target region identified during a treatment planning phase;
responsive to determining that the current position of the target region corresponds to any one of a plurality of predetermined positions of the target region identified during a treatment planning phase:
selecting a treatment sub-plan from a first set of treatment sub-plans, the first set of treatment sub-plans each corresponding to a different predetermined position of the target region, wherein the selected treatment sub-plan corresponds to the current position of the target region;
responsive to determining that the current position of the target region does not correspond to one of the plurality of predetermined position of the target region:
determining that the current position of the region corresponds to a location within a predetermined volume of space encapsulating the plurality of predetermined positions; and
selecting a treatment sub-plan from a second set of treatment sub-plans, the second set of treatment sub-plans each corresponding to a different location within the predetermined volume of space, wherein the selected treatment subplan corresponds to the current position of the target region.
